# EUROPÄISCHE PATENTSCHRIFT

(11) **EP 0 568 497 B1**
(45) Veröffentlichungstag und Bekanntmachung des Hinweises auf die Patenterteilung: **07.07.1999**
(21) Anmeldenummer: 93810288.6
(22) Anmeldetag: 21.04.1993
(51) Int. Cl.: C07D 209/44, C09B 57/04

(54) **Chromogene Methylenpyrroline**
Chromogene methylenepyrrolins
Méthylènepyrrolines chromogènes

(30) Priorität: 30.04.1992 CH 138892
(43) Veröffentlichungstag der Anmeldung: 03.11.1993
(73) Patentinhaber: Ciba Specialty Chemicals Holding Inc., 4057 Basel (CH)
(72) Erfinder: Bedekovic, Davor, Dr., CH-4105 Biel-Benken (CH)

(56) Entgegenhaltungen:
- EP-A- 15 570
- EP-A- 17 132
- EP-A- 36 388
- EP-A- 61 094
- EP-A- 209 028
- EP-A- 298 037
- PATENT ABSTRACTS OF JAPAN vol. 011, no. 317 (C-452)15. Oktober 1987

## Beschreibung

Die vorliegende Erfindung betrifft chromogene Methylenpyrroline sowie Verfahren zu ihrer Herstellung und ihre Verwendung.

In den Druckschraiften EP-A-0,015,570, EP-A-0,061,094, EP-A-0,209,028, EP-A-0,298,037, EP-A-0,036,388,Patent Abstracts of Japan 001, 317 (C-452) (1987) werden Isoindolinverbindungen offenbart, die in photographischen Aufzeichnungsmaterialien Verwendung finden. In der EP-A-0,017,132 werden Isoindole offenbart, die als Farbstoffe für synthetische Fasermaterialien verwendet werden.

Die erfindungsgemässen chromogenen Methylenpyrrolinverbindungen haben die allgemeine Formel 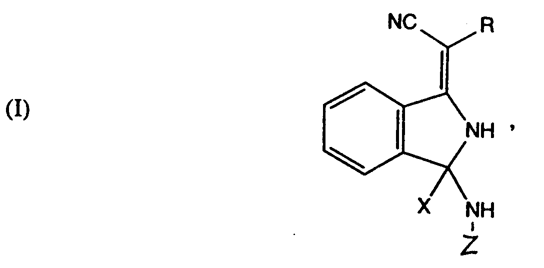 worin
- X: Indolyl, einen unsubstituierten oder bis zu dreifach gleich oder verschieden durch Halogen, Cyano, C₁-C₁₂-Alkyl, C₅-C₆-Cycloalkyl, C₁-C₈-Acyl, -NR₁R₂, -OR₃ oder -SR₃ substituierten Phenylrest,
- R: CN oder durch Hydroxy, Halogen, Nitro, Cyano, Acyl mit 1 bis 8 Kohlenstoffatomen, unsubstituiertes oder durch Halogen, Hydroxy, Cyano oder C₁-C₁₂-Alkoxy substituiertes C₁-C₁₂-Alkyl, oder C₁-C₁₂-Alkoxy,
- Z: C₁-C₁₂-Alkyl-CO- oder unsubstituiertes oder durch Halogen, Nitro, C₁-C₁₂-Alkyl oder C₁-C₁₂ Alkoxy substituiertes Benzoyl,
- R₁, R₂ und R₃,: unabhängig voneinander, Wasserstoff, unsubstituiertes oder durch Halogen, Hydroxy, Cyano oder C₁-C₁₂-alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₁₂-Alkyl, C₁-C₁₂-Alkoxy, C₁-C₁₂-Alkoxycarbonyl, -NX'X'' oder 4-NX'X''-Phenylamino ringsubstituiertes Phenylalkyl oder Phenyl,
- X' und X'',: unabhängig voneinander, Wasserstoff, C₁-C₁₂-Alkyl, Cyclohexyl, Benzyl oder Phenyl oder
- R₁ und R₂: zusammen mit dem sie verbindenden Stickstoffatom einen fünf- oder sechsgliedrigen, vorzugsweise gesättigten, heterocyclischen Rest
bedeuten.

Ist in Formel (1) X ein heteroaromatischer Rest, so ist dieser vorzugsweise über ein Kohlenstoffatom des Heteroringes an das Kohlenstoffatom der Pyrrolinverbindung gebunden.

Die Alkyl- und Alkenylreste können geradkettig oder verzweigt sein. Beispiele hierfür sind Methyl, Ethyl, n-Propyl, Isopropyl, n-Butyl, 1-Methylbutyl, sek.Butyl, t.Butyl, Amyl, Isopentyl, n-Hexyl, 2-Ethylhexyl, Isooctyl, n-Octyl, 1,1,3,3-Tetramethylbutyl, Nonyl, Isononyl, 3-Ethylheptyl, Decyl oder n-Dodecyl bzw. Vinyl, Allyl, 2-Methylallyl, 2-Ethylallyl, 2-Butenyl oder Octenyl. C₁-C₁₂-Alkenylreste sind über ein gesättigtes Kohlenstoffatom an Heteroatome, insbesondere Stickstoff, gebunden.

Als Substituent des Restes X ist "Acyl" besonders Formyl, C₁-C₁₂-Alkylcarbonyl, wie z.B. Acetyl oder Propionyl, oder Benzoyl. Weitere Acylreste können C₁-C₁₂-Alkylsulfonyl, wie z.B. Methylsulfonyl oder Ethylsulfonyl sowie Phenylsulfonyl sein. Benzoyl und Phenylsulfonyl können durch Halogen, Methyl, Methoxy oder Ethoxy substituiert sein.

C₁-C₁₂- Alkyl, C₁-C₁₂-Alkoxy und C₁-C₁₂-Alkylthio stellen solche Gruppen oder Gruppenbestandteile dar, die 1 bis 12, vorzugsweise 1 bis 6, insbesondere 1 bis 3 Kohlenstoffatome aufweisen. Beispiele für derartige Gruppen sind Methyl, Ethyl, n-Propyl, Isopropyl, n-Butyl, sek.Butyl, tert.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.

Halogen bedeutet beispielsweise Fluor, Brom oder vorzugsweise Chlor.

Bevorzugte IndolylReste vor allem 3-Indolylreste, wie z.B. 2-Phenylindol-3-yl-, 2-Methylindol-3-yl, N-C₁-C₈-Alkyl-2-methylindol-3-yl-, N-C₂-C₄-Alkanoyl-2-methylindol-3-yl, 2-Phenylindol-3-yl- oder N-C₁-C₈-Alkyl-2-phenylindol-3-yl-Reste.

Als aromatischer Rest stellt X vorzugsweise einen substituierten Phenylrest der Formel 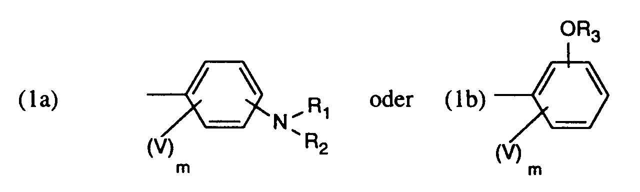 dar,worin
- R₁, R₂ und R₃,: unabhängig voneinander, Wasserstoff, unsubstituiertes oder durch Halogen, Hydroxy, Cyano oder C₁-C₁₂-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₁₂-Alkyl C₁-C₁₂-Alkoxy, C₁-C₁₂-Alkoxycarbonyl, -NX'X'' oder 4-NX'X''-Phenylamino ringsubstituiertes Phenylalkyl oder Phenyl,
- X' und X'',: unabhängig voneinander, Wasserstoff, C₁-C₁₂-Alkyl, Cyclohexyl, Benzyl oder Phenyl oder
- R₁ und R₂: zusammen mit dem sie verbindenden Stickstoffatom einen fünf- oder sechsgliedrigen, vorzugsweise gesättigten, heterocyclischen Rest,
- V: Hydroxy; Halogen, C₁-C₁₂-Alkyl, C₁-C₁₂-Alkoxy, C₅-C₇-Cycloalkoxy, C₁-C₁₂-Acyloxy, unsubstituiertes oder im Phenylrest durch Halogen, Cyano, C₁-C₁₂-Alkyl oder C₁-C₁₂-Alkoxy substituiertes Benzyl, Phenyl, Benzyloxy, Phenoxy oder beim Phenylrest der Formel (1a) auch die Gruppe -NT₁T₂
- T₁ und T₂,: unabhängig voneinander, Wasserstoff, C₁-C₁₂-Alkyl, C₅-C₁₀-Cycloalkyl, unsubstituiertes oder durch Halogen, Cyano, C₁-C₁₂-Alkyl oder C₁-C₁₂-Alkoxy substituiertes Benzyl, oder Acyl mit 1 bis 8 Kohlenstoffatomen und
- T₁: auch unsubstituiertes oder durch Halogen, Cyano, C₁-C₁₂-Alkyl oder C₁-C₁₂-Alkoxy substituiertes Phenyl, und
- m: 0, 1 oder 2,
bedeutet.

Die Substituenten -NR₁R₂ und -OR₃ befinden sich vorzugsweise in Para-Stellung zur Verknüpfungsstelle. V steht vorzugsweise in Ortho-Stellung zur Verknüpfungsstelle.

Als Alkyl stellen R₁, R₂ und R₃ beispielsweise die für Alkylreste oben aufgezählten Substituenten dar.

Sind die Alkylreste in R₁, R₂ und R₃ substituiert, so handelt es sich vor allem um Cyanoalkyl, Halogenalkyl, Hydroxyalkyl, Alkoxyalkyl, jeweils vorzugsweise mit insgesamt 2 bis 8 Kohlenstoffatomen, wie z.B. 2-Cyanoethyl, 2-Chlorethyl, 2-Hydroxyethyl, 2-Methoxyethyl, 2-Ethoxyethyl, 2,3-Dihydroxypropyl, 2-Hydroxy-3-chlorpropyl, 3-Methoxypropyl, 4-Methoxybutyl oder 4-Propoxybutyl.

Beispiele für Cycloalkyl in der Bedeutung von R₁, R₂, R₃, T₁ und T₂ sind Cyclopentyl, Cycloheptyl oder vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Als Aralkyl bzw. Phenylalkyl können R₁, R₂ und R₃ Phenethyl, Phenylisopropyl oder vor allem Benzyl sein.

Bevorzugte Substituenten in der Phenylalkyl- und Phenylgruppe der von R₁, R₂ und R₃ sind z.B. Halogen, Cyano, Methyl, Trifluormethyl, Methoxy oder Carbomethoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorphenyl, Methoxyphenyl, 2,6-Dimethylphenyl, Trifluormethylphenyl oder Carbomethoxyphenyl.

Der Acyloxyrest in V ist beispielsweise Formyloxy, C₁-C₁₂-Alkylcarbonyloxy, wie z.B. Acetyloxy oder Propionyloxy, oder Benzoyloxy. Als C₁-C₁₂-Alkoxyrest kann V eine geradkettige oder verzweigte Gruppe sein, wie z.B. Methoxy, Ethoxy, Isopropoxy, n-Butoxy, tert.Butoxy, Amyloxy, 1,1,3,3-Tetramethylbutoxy, n-Hexyloxy, n-Octyloxy oder Dodecyloxy. Beispiele für Cycloalkoxy in der Bedeutung von V sind Cyclopentyloxy, Cycloheptyloxy oder vorzugsweise Cyclohexyloxy.

Wenn das Substituentenpaar (R₁ und R₂) zusammen mit dem gemeinsamen Stickstoffatom einen heterocyclischen Rest darstellt, so ist dieser beispielsweise Pyrrolidino, Piperidino, Pipecolino, Morpholino, Thiomorpholino, Piperazino, N-Alkylpiperazino, wie z.B. N-Methylpiperazino, N-Phenylpiperazino oder N-Alkylimidazolino. Bevorzugte gesättigte heterocyclische Reste für -NR₁R₂ sind Pyrrolidino, Piperidino oder Morpholino.

Dye Substituenten R₁ und R₂ sind vorzugsweise Cyclohexyl, Benzyl, Phenethyl, Cyano-C₁-C₁₂-Alkyl z.B. β-Cyanoethyl oder in erster Linie C₁-C₁₂-Alkyl, wie z.B. Methyl, Ethyl, n-Pentyl oder vor allem n-Butyl. -NR₁R₂ ist bevorzugt auch Pyrrolidinyl. R₃ ist vorzugsweise C₁-C₁₂-Alkyl oder Benzyl.

V kann vorteilhafterweise Wasserstoff, Hydroxy, Halogen, C₁-C₁₂-Alkyl, C₁-C₈-Alkoxy, Benzyloxy, wie z.B. Methyl, Methoxy, Ethoxy, Isopropoxy oder n-Butoxy, oder die Gruppe -NT₁T₂ sein, wobei von den Resten T₁ und T₂ einer vorzugsweise C₁-C₈-Acyl oder C₁-C₁₂-Alkyl und der andere Wasserstoff oder C₁-C₁₂-Alkyl ist. Der Acylrest ist in diesem Falle besonders C₁-C₁₂-Alkylcarbonyl, wie z.B. Acetyl oder Propionyl. Vorzugsweise ist V Acetylamino, Dimethylamino, Diethylamino, Hydroxy, Benzyloxy oder besonders C₁-C₁₂-Alkoxy und vor allem Ethoxy oder n-Butoxy.

Acylreste im Sinne von Z sind am Stickstoffatom thermisch, zweckmässigerweise oberhalb 100°C, abspaltbare Substituenten.

Vorzugsweise ist Z eine Acylgruppe der Formel Y'-CO-, worin Y' C₃-C₈-Alkyl oder Phenyl bedeutet. Alkyl kann geradkettig oder verzweigt sein.

Der Substituent R als Phenylcarbamoyl steht insbesondere für einen Rest der Formel 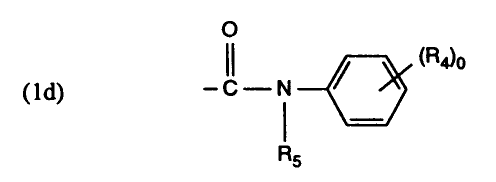 worin
- R₄: unabhängig voneinander Hydroxy, Halogen, Nitro, Cyano, Acyl mit 1 bis 8 Kohlenstoffatomen, unsubstituiertes oder durch Halogen, Hydroxy, Cyano oder C₁-C₁₂-Alkoxy substituiertes C₁-C₁₂-Alkyl oder C₁-C₁₂-Alkoxy,
- R₅: Wasserstoff oder C₁-C₁₂-Alkyl und
- o: 0,1, 2 oder 3,
bedeutet.

Bevorzugt sind Isoindoline der Formel 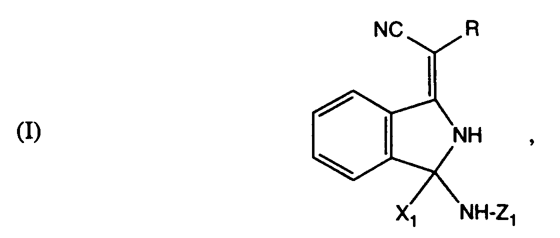 worin
- Z₁: C₁-C₁₂-Alkyl-CO- oder unsubstituiertes oder durch Halogen, Nitro, C₁-C₁₂-Alkyl oder, C₁-C₁₂-Alkoxy substituiertes Benzoyl und
- X₁: einen 3-Indolylrest der Formel 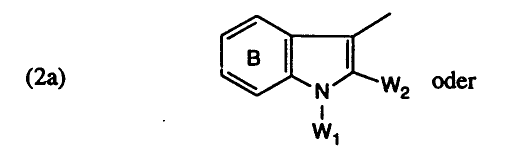 einen substituierten Phenylrest der Formel 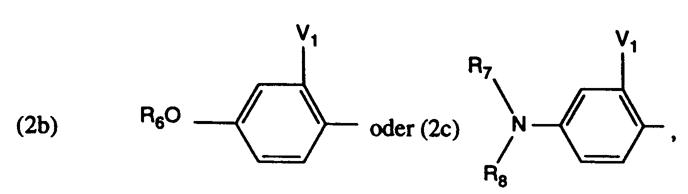
- W₁: Wasserstoff, unsubstituiertes oder durch Cyano oder C₁-C₁₂-Alkoxy substituiertes C₁-C₈-Alkyl, Acetyl Propionyl oder Benzyl,
- W₂: Wasserstoff, C₁-C₁₂-Alkyl, vor allem Methyl, oder Phenyl,
- R₆, R₇ und R₈,: unabhängig voneinander, je unsubstituiertes oder durch Hydroxy, Cyano oder C₁-C₁₂-Alkoxy substituiertes Alkyl mit höchstens 12 Kohlenstoffatomen, C₅-C₆-Cycloalkyl, Benzyl, Phenethyl oder Phenyl und R₆ auch Wasserstoff, oder (R₅ und R₆) zusammen mit dem sie verbindenden Stickstoffatom Pyrrolidino, Piperidino oder Morpholino,
- V₁: Wasserstoff, Hydroxy, Halogen, C₁-C₁₂-Alkyl, C,-C₈-Alkoxy, Benzyloxy oder die Gruppe -NT₃T₄,
- T₃ und T₄,: unabhängig voneinander, je Wasserstoff, C₁-C₁₂-Alkyl, C₁-C₁₂-Alkylcarbonyl oder unsubstituiertes oder durch Halogen, Methyl oder Methoxy substituiertes Benzoyl
- R: Cyano oder einen unsubstituierten oder substituierten Rest der Formel 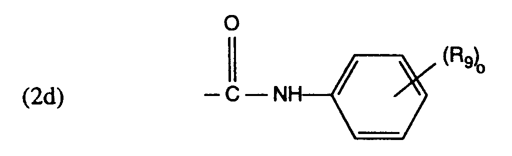
- R₉: Halogen oder C₁-C₁₂-Alkyl,
- o: 0, 1 oder 2 bedeutet und
- B: nicht substituiert oder durch Halogen, C₁-C₁₂-Alkyl, wie Methyl oder Isopropyl oder durch Di-C₁-C₁₂-Alkylamino, wie Dimethylamino substituiert ist.
Unter den Verbindungen der Formel (I) sind die Isoindolinverbindungen, in denen X₁ einen 3-Indolylrest der Formel (2a), W₁ Wasserstoff oder C₁-C₈-Alkyl, W₂ Methyl oder Phenyl bedeuten, und Z₁ C₃-C₈-Alkylcarbonyl darstellen, besonders bevorzugt.

Hervorzuheben sind
a) Dicyanoisoindoline der Formel 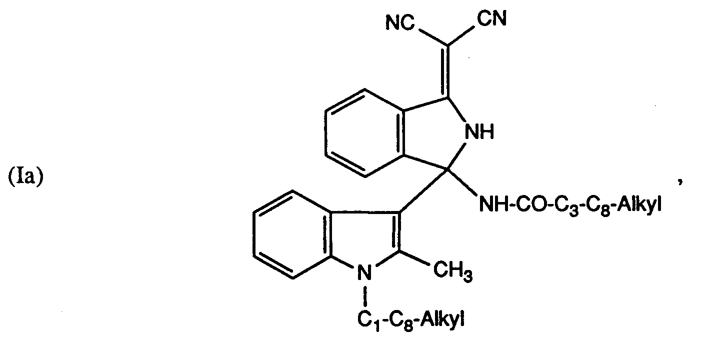
b) Dicyanoisoindoline der Formel 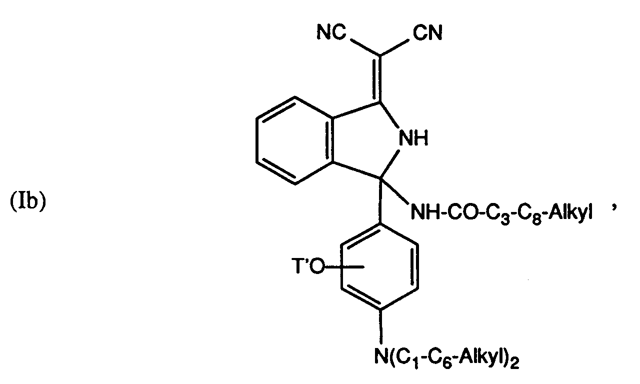 worin T' Wasserstoff oder C₁-C₆-Alkyl bedeutet,
c) Dicyanoisoindoline der Formel 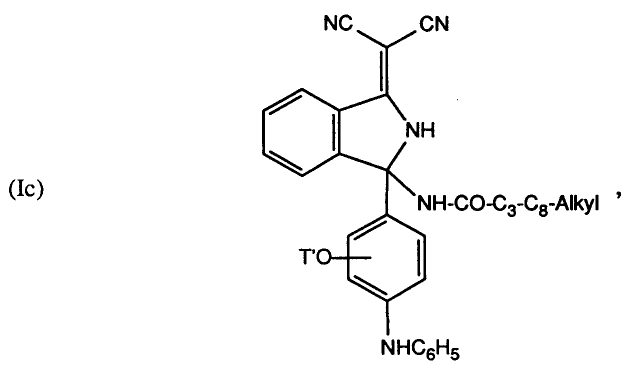 worin T' Wasserstoff oder C₁-C₆-Alkyl bedeutet,
d) Isoindoline der Formel 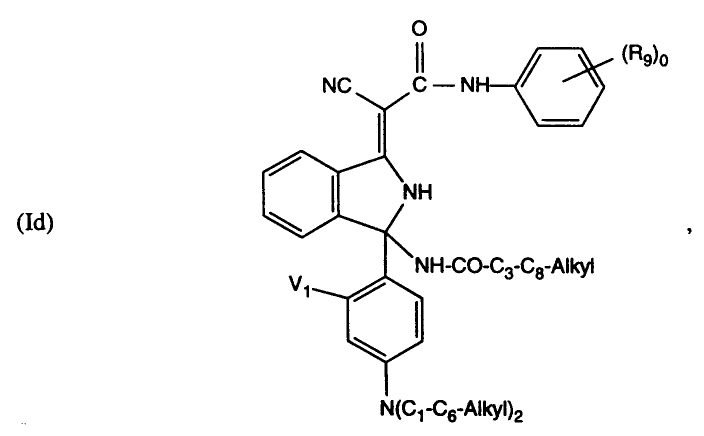 worin
   - R₉: Halogen oder C₁-C₁₂-Alkyl,
   - o: 0, 1 oder 2 und
   - V₁: Wasserstoff oder Hydroxy
   bedeutet,
   oder
e) Isoindoline der Formel 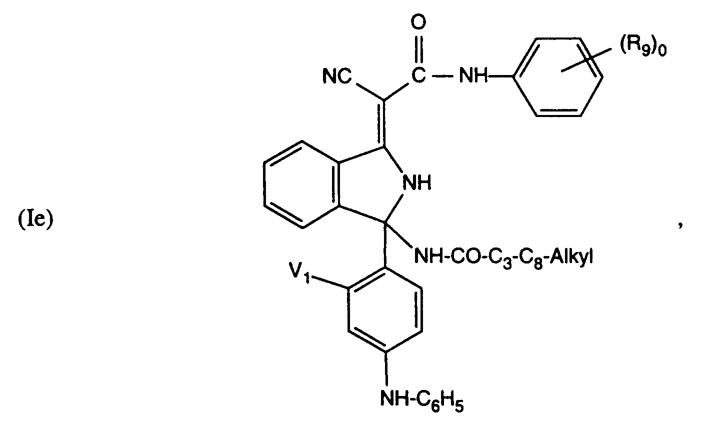 worin
   - R₉: Halogen, C₁-C₁₂-Alkyl oder C₁-C₁₂-Alkoxy,
   - o: 0, 1 oder 2 und
   - V₁: Wasserstoff oder Hydroxy
   bedeutet.

Die erfindungsgemässen Pyrroline der Formeln (I), (Ia), (Ib), (Ic), (Id) oder (Ie) werden dadurch hergestellt, dass man eine entsprechende aromatische Verbindung

X-H (II)

worin
- X: wie zuvor definiert ist, an ein Pyrrolinderivat der Formel 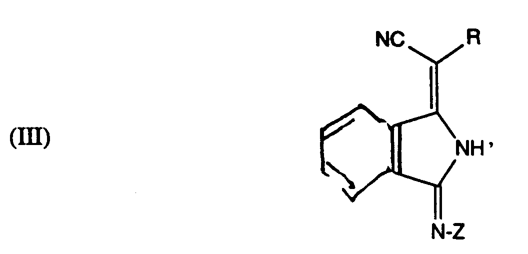 worin
- R und Z: wie zuvor definiert sind, anlagert.

Die Anlagerung wird bei einer Temperatur von 15 bis höchstens 70°C, vorzugsweise bei Raumtemperatur (15-30°C) durchgeführt. Zweckmässigerweise erfolgt sie in organischen Lösungsmitteln und gegebenenfalls unter Einwirkung saurer Kondensationsmittel.

Geeignete Lösungsmittel, die das Reaktionsmedium bilden, sind Alkohole, wie z.B. Methanol, Ethanol, Propanol, Ethylenglykolmonomethyl- oder -ethylether, Ketone, z.B. Aceton, Butanon oder Methylisopropylketon, Dimethylformamid, Dimethylsulfoxid oder Nitrile aliphatischer Monocarbonsäuren, wie z.B. Acetonitril, Propionitril oder Butyronitril, cycloaliphatische oder aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Monochlorbenzol, Monochlortoluol oder Dichlorbenzol oder cyclische Ether, wie z.B. Dioxan oder Tetrahydrofuran.

Bevorzugte Lösungsmittel sind Methanol, Aceton, Methylethylketon und Toluol sowie Mischungen davon.

Als saure Kondensationsmittel können beispielsweise Phosphorsäure, Schwefelsäure, Salzsäure, Essigsäure, Trifluoressigsäure, Propionsäure und vor allem p-Toluolsulfonsäure, Fluorbenzoesäure, Nitrobenzoesäure, Benzoesäure und Trichloressigsäure verwendet werden.

Die Reaktionsdauer der Anlagerung hängt von dem Kondensationsmittel, dem Lösungsmittel und der verwendeten Anlagerungsverbindung ab und liegt in der Regel zwischen 2 und 10 Stunden, vorzugsweise 2,5 und 6 Stunden.

Nach der Anlagerung wird die erhaltene chromogene Dicyanmethylenpyrrolinverbindung auf übliche Weise isoliert, beispielsweise indem man das Lösungsmittel durch Destillation entfernt, danach die ausgefallene Pyrrolinverbindung abfiltriert und trocknet. Falls erforderlich, kann die Pyrrolinverbindung durch Umkristallisieren z.B. in Toluol gereinigt werden.

Besonders bevorzugte Anlagerungsverbindungen sind Aniline, wie Kresidine, Phenetidine, 3-Butoxyaniline, N,N-Dialkylaminophenole, sowie 2-Niederalkylindole oder 2-Phenylindole, die jeweils durch C₁-C₈-Alkyl N-substituiert sein können.

Spezifische Beispiele für Anlagerungsverbindungen der Formel (II) sind N,N-Dimethylanilin, N,N-Diethylanilin, N,N-Dibenzylanilin, N,N-Dibutylanilin, N-Methyl-N-Cyclohexylanilin, 3-Methoxy-N,N-Dimethylanilin, 3-Ethoxy-N,N-Diethylanilin, 3-Ethoxy-N,N-di-n-pentylanilin, 3-n-Butoxy-N,N-di-n-butylanilin, 3-Acetyl-amino-N,N-dipropylanilin, 2-Methyl-5-hydroxy-N-ethylanilin (3-Ethylamino**-**4**-**kresol), β-Hydroxydiphenylamin, 4-Ethoxydiphenylamin, 3-Ethoxy-N,N-dimethylanilin, 3-Hydroxy-N,N-dimethylanilin, 3-Hydroxy-N,N-Diethylanilin, 3-Hydroxy-N,N-di-n-butylanilin, 2-Methylindol, 2-Phenylindol, 1,2-Dimethylindol, 1-Ethyl-2-methylindol, 1-n-Butyl-2-methylindol, 1-n-Octyl-2-methylindol, N-Butylcarbazol, 3-Methyl-6-dimethylaminoindol, 3-Methyl-6-ethoxyindol, 1-Ethyl-3-methyl-6-ethoxyindol.

Die als Ausgangsstoffe benötigten Verbindungen der Formel (III), in denen R Cyano bedeutet, werden beispielsweise durch Umsetzen von 1,3-Diiminopyrrolen, wie etwa 1,3-Diiminoisoindolinen, mit Malonsäuredinitril und anschliessender Einführung des Restes Z durch Acylierung hergestellt. Sowohl die Umsetzung mit Malonsäuredinitril als auch die Acylierung erfolgen in bekannter Weise.

Die Verbindungen der Formel (III) sind aus der EP-A-0,017,132 bekannt und wertvolle Ausgangsverbindungen für die erfindungsgemässen Farbbildner der Formel (I).

Die erfindungsgemässen Verbindungen der Formeln (I), (Ia), (Ib), (Ic) oder (Id) eignen sich insbesondere als Farbbildner in einem thermoreaktiven Aufzeichnungsmaterial. Sie zeichnen sich dadurch aus, dass sie für die Bildung der Farbe keine konventionellen sauren Farbentwickler, wie z.B. Tone, Phenole, Zinksalicylate oder Phenolharze benötigen. Erfindungsgemässe Verbindungen können auch als Transferfarbstoffe z.B. für den Thermodiffusionstransferprozess verwendet werden.

Der Farbton des im wärmeempfindlichen Aufzeichnungsmaterial erhaltenen Bildes kann je nach der Bedeutung von X rot, violett, blau oder braun sein. Die erhaltenen Bilder sind scharf und klar.

Die wärmeempfindlichen Aufzeichnungsmaterialien werden beispielsweise zum Aufzeichnen von Informationen, z.B. in elektronischen Rechnern, Druckern, Faksimile- oder Kopiermaschinen, Fernschreibern oder in medizinischen oder technischen Aufzeichnungsgeräten und Messinstrumenten, wie z.B. Elektrocardiographen, oder zur Herstellung von Etiketten oder Bar Codes verwendet.

Die Bilderzeugung (Markierung) kann auch manuell mit einer erhitzten Feder erfolgen. Eine weitere Einrichtung zur Erzeugung von Markierungen mittels Wärme sind Laserstrahlen.

Die wärmeempfindlichen Aufzeichnungssysteme enthalten zweckmässigerweise mindestens einen Schichtträger, wie z.B. Papier, synthetisches Papier, eine Kunstfolie und eine oder mehrere darauf ausgebildete temperaturempfindliche Schichten, welche die Dicyanmethylenverbindung der Formeln (I), (Ia), (Ib), (Ic) oder (Id) enthalten. Falls erforderlich, können erfindungsgemässe, wärmeempfindliche Aufzeichnungsmaterialien Aktivatoren oder Sensibilisatoren enthalten.

Vorzugsweise werden zur Herstellung des thermoreaktiven Aufzeichnungsmaterials schmelzbare, filmbildende Bindemittel verwendet. Diese Bindemittel sind normalerweise wasserlöslich, während erfindungsgemässe Dicyanmethylenverbindungen in Wasser unlöslich sind Das Bindemittel sollte in der Lage sein, die Dicyanmethylenverbindung bei Raumtemperatur zu dispergieren und zu fixieren.

Bei Einwirkung von Wärme löst sich aus der als Farbbildner auftretenden Dicyanmethylenverbindung die Verbindung Z-NH₂ ab, und es bildet sich eine Farbe.

Wasserlösliche oder mindestens in Wasser quellbare Bindemittel sind z.B. hydrophile Polymerisate, wie Polyvinylalkohol, Alkalimetall-Polyacrylate, Hydroxyethylcellulose, Methylcellulose, Carboxymethylcellulose, Polyacrylamid, Polyvinylpyrrolidon, carboxylierte Butadien-Styrolocopolymerisate, Gelatine, Stärke oder veretherte Maisstärke.

Die thermoreaktiven Schichten können weitere Zusätze enthalten. Zur Verbesserung des Weissgrades oder der Thermokopfeignung und deshalb zur Erleichterung des Bedruckens der Papiere und zur Verhinderung des Festklebens der erhitzten Feder können diese Schichten z.B. Antioxidantien, UV-Absorber, Lösungshilfen, Talk, Titandioxid, Zinkoxid, Aluminiumoxid, Aluminiumhydroxid, Calciumcarbonat (z.B. Kreide), Magnesiumcarbonat, Tone oder auch organische Pigmente, wie z.B. Harnstoff**-**Formaldehyd-Polymerisate enthalten. Zur Farbbildung nur innerhalb eines begrenzten Temperaturbereichs können Substanzen wie Harnstoff, Thioharnstoff, Diphenylthioharnstoff, Acetamid, Acetanilid, Benzolsulfanilid, Stearinsäuraeamid, Triphenylmethan, p-Benzylbiphenyl, Phthalsäureanhydrid, Metallstearate, wie z.B. Zinkstearat, Phthalsäurenitril, Dimethylterephthalat oder andere entsprechende, schmelzbare Produkte, welche die Z-NH₂-Bildung induzieren, zugesetzt werden. Bevorzugt enthalten die thermographischen Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin. Gegebenenfalls können die Aufzeichnungsmaterialien auch alkalische Materialien, wie z.B. Hydroxide oder Carbonate von Alkalimetallen oder vorzugsweise offene oder cyclische organische Basen wie Amine, Alkanolamine, Guanidine, Pyridine oder Imidazolderivate enthalten.

In den folgenden Beispielen beziehen sich die angegebenen Prozentsätze, wenn nichts anderes angegeben ist, auf das Gewicht. Teile sind Gewichtsteile.

### A. Herstellungsbeispiele

### Beispiel 1: 1-Dicyanomethylen-3-(2,2-dimethylpropionylamino)-3(2-methyl-1-n-octyl-indol-3-yl)-isoindolin

2,8 g 1-Dicyanmethylen-3-dimethylpropionyliminoisoindolin werden zusammen mit 2,43 g 1-n-Octyl-2-methylindol und 0,2 g p-Toluolsulfonsäure in 15 ml Toluol und 10 ml Aceton bei Raumtemperatur 6 Stunden gerührt. Hierauf wird das Lösungsmittelgemisch eingeengt, wobei Niederschlagsbildung erfolgt. Der Niederschlag wird abfiltriert, mit Toluol gewaschen und getrocknet. Man isoliert 3 g der Verbindung der Formel 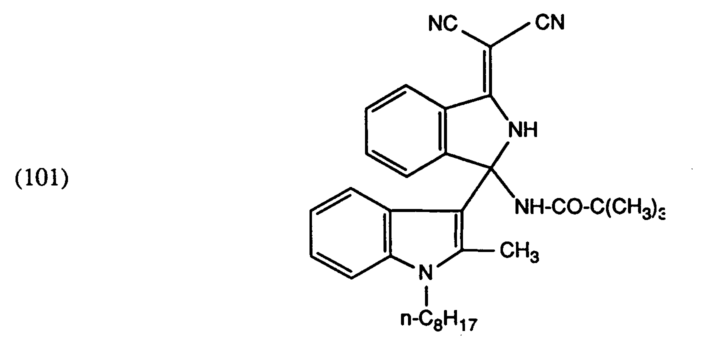 als farblose Kristalle mit einem Schmelzpunkt von 178-179°C. In der Thermographie entwickelt dieser Farbbildner eine rotviolette Markierung.

Analog zu Beispiel 1 werden die Verbindungen der Tabelle 1 erhalten.

Entsprechend der Ausführungsform im Beispiel 1 werden bei Verwendung der entsprechenden Reaktionskomponenten und Anlagerungstemperatur die in der nachfolgenden Tabelle angegebenen Dicyanmethylenisoindoline erhalten. In der letzten Spalte der Tabelle ist die in der Thermographie erhaltene Farbe angegeben.

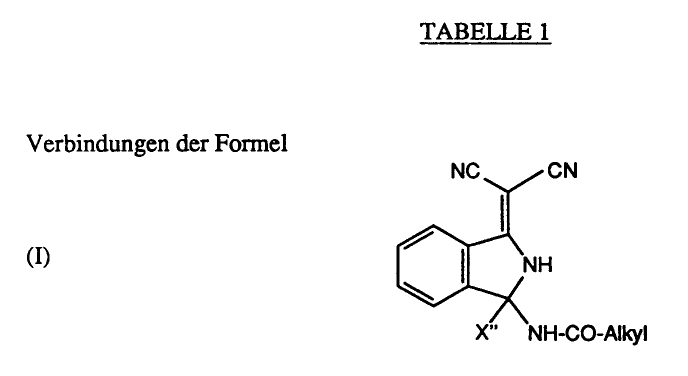

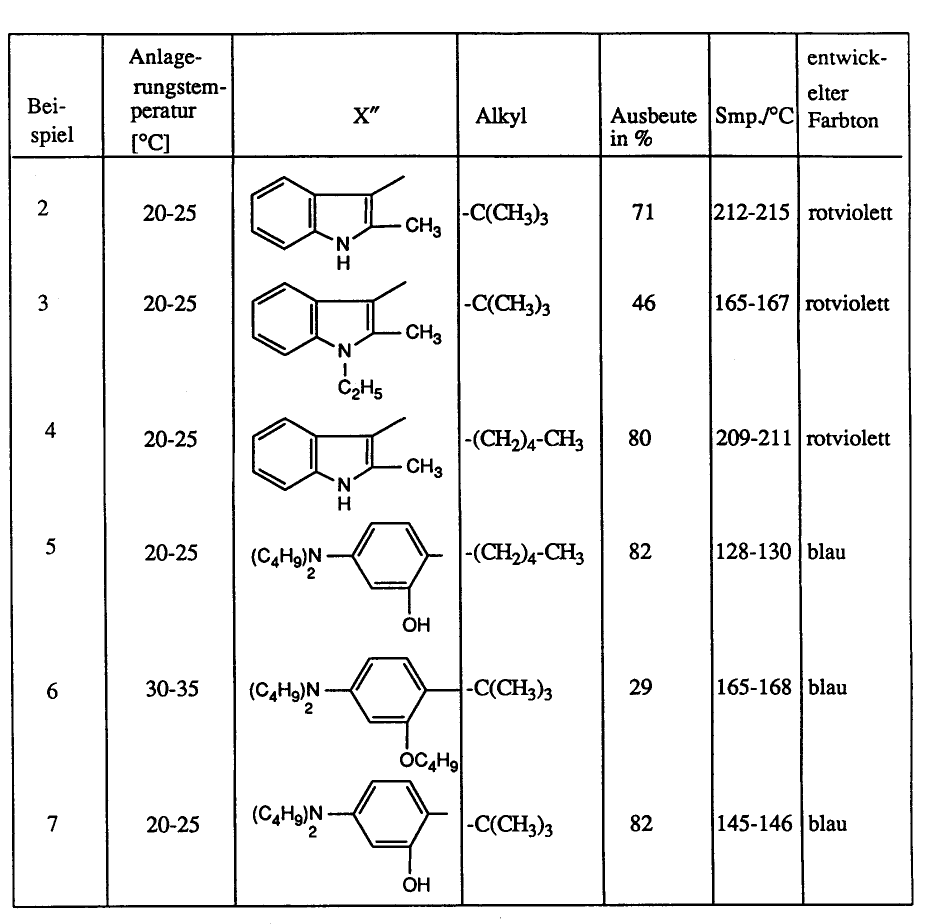

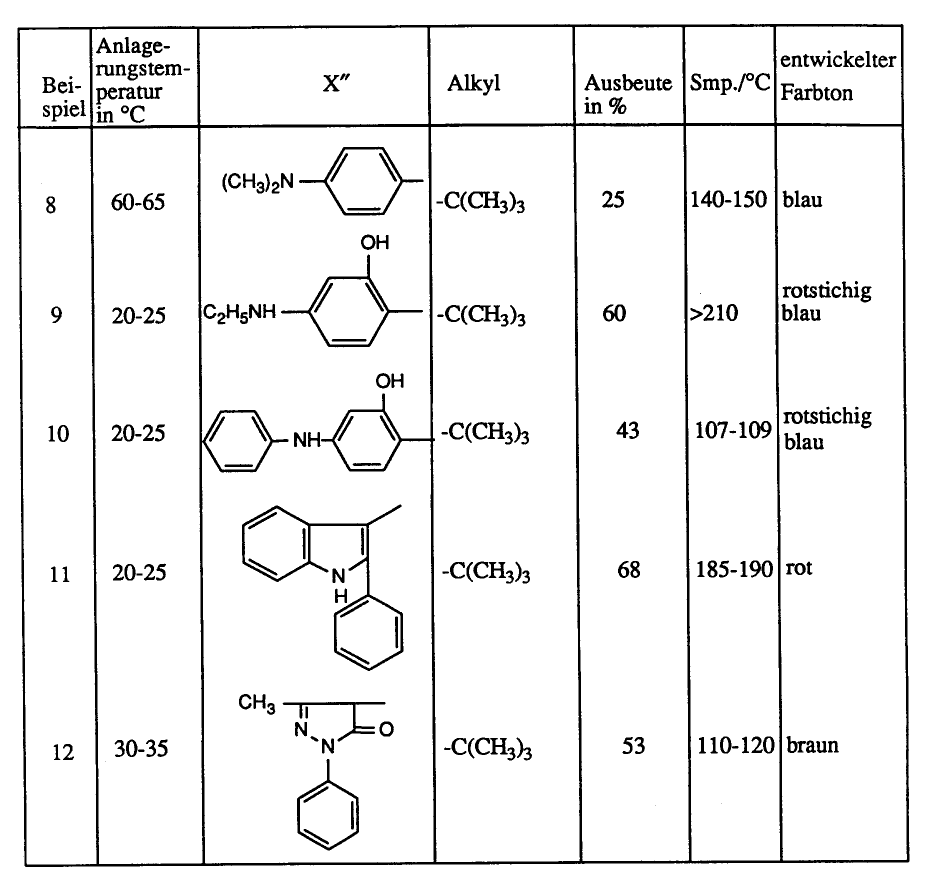

### Beispiel 13: 1-(α-Cyano-α-[3,4-dichlorphenylaminocarbonyl]-methylen)-3(2,2-dimethyl-propionylamino)-3(4-dibutylamino-2-hydroxyphenyl)-isoindolin

2,23g 1-(α-Cyano-α-[3,4-dichlorphenylaminocarbonyl]-methylen)-3(2,2-dimethylpropionylimino)-isoindol werden zusammen mit 1,02 g Dibutylaminophenol und 0,1 g Trichloressigsäure in 20 ml Toluol bei Raumtemperatur 3 Stunden gerührt. Anschliessend wird noch 1 Stunde auf 35 - 40° C erwärmt, bis kein Edukt mehr nachgewiesen werden kann (Dünnschichtchromatographie). Es wird von dem ausgefallenen Produkt abfiltriert, mit etwas Toluol nachgewaschen und die so erhaltenen Kristalle in Aceton aufgenommen und mit Aktivkohle gereinigt. Die Aceton-Phase wird eingeengt, die ausgefallenen Kristalle mit n-Hexan gewaschen und im Vakuum bei Raumtemperatur getrocknet.

Man isoliert 2,0 g der Titelverbindung der Formel 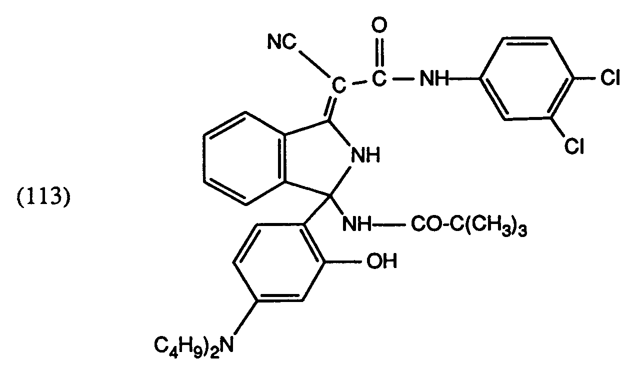 als beigefarbene Kristalle vom Smp. 168 - 170° C.

Analog zu den vorstehenden Herstellungsbeispielen können die Verbindungen der Tabelle 2 hergestellt werden:

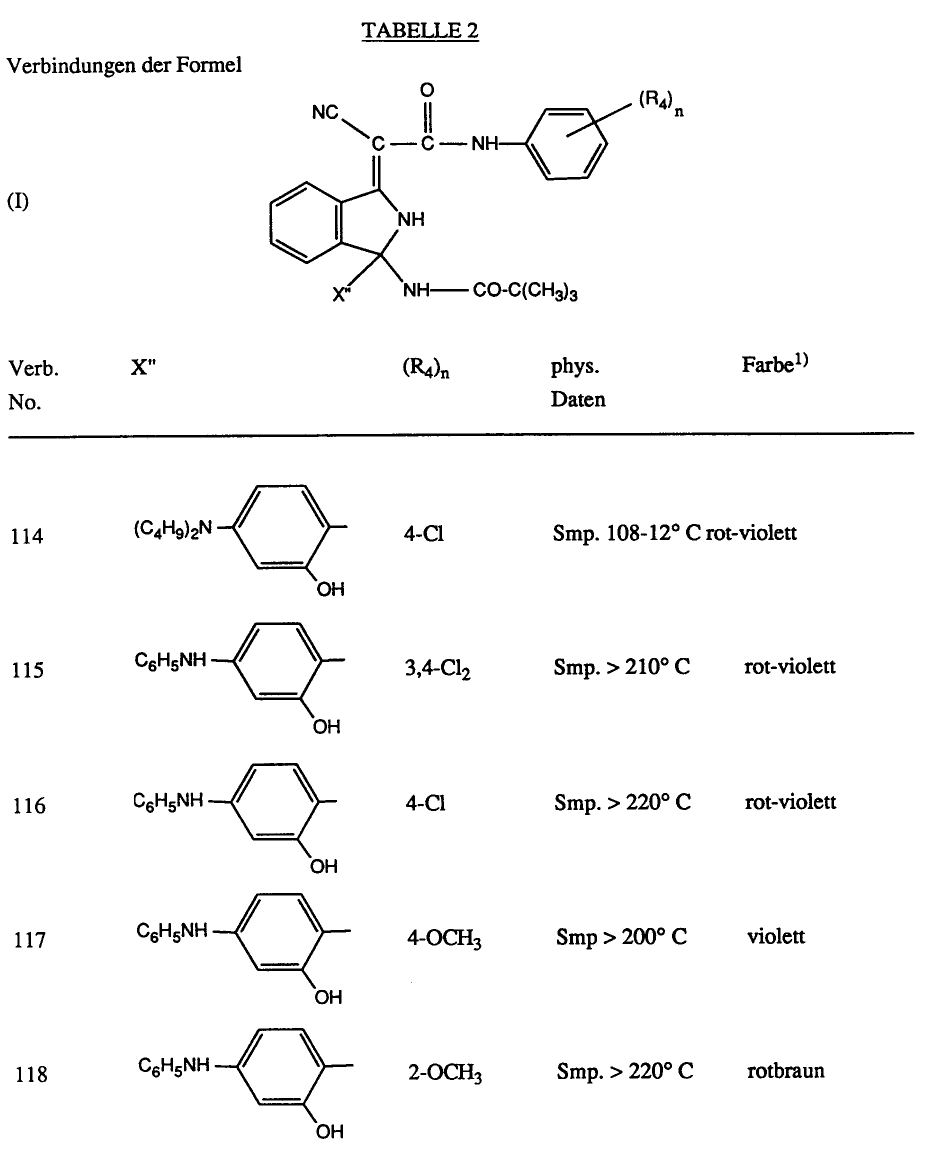

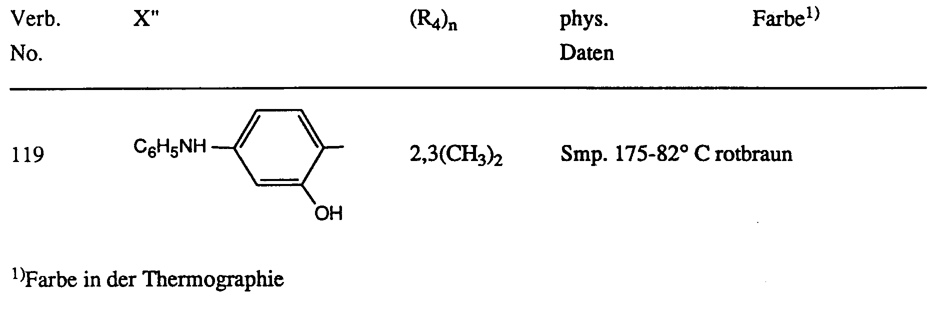

### B. Anwendungsbeispiele

### Beispiel 20: Zur Herstellung einer Dispersion werden

- 2 g: der Dicyanmethylenisoindolinverbindung (107),
- 7 g: einer 10%igen wässrigen Lösung von Polyvinylalkohol und
- 4 g: Wasser
in einer Perlmühle während 4 bis 5 Stunden zu einer Korngrösse von 24 µm gemahlen. Die Dispersion wird von den Perlen getrennt und 18 Stunden gelagert. Die Dispersion wird dann auf ein Streichpapier mit einem Flächengewicht von 50 g/m² mit einem Rakel aufgetragen. Die aufgebrachte Streichmischung entspricht 2 g/m² Trockengewicht.

Durch Verwendung des Papiers in einem Faksimile-Gerät (Infotec 6510) entwickelt sich eine blaue Farbe.

### Beispiel 21: Zur Herstellung einer Dispersion A werden

- 2 g: der Dicyanmethylenisoindolinverbindung (101),
- 7 g: einer 10%-igen wässrigen Lösung von Polyvinylalkohol und
- 4 g: Wasser
in einer Perlmühle während 4 bis 5 Stunden zu einer Korngrösse von 2-4 µm gemahlen. Die Dispersion wird von den Perlen getrennt und 18 Stunden gelagert.

### Zur Herstellung einer Dispersion B werden

- 5 g: Triphenylmethan und
- 12 g: einer 10 % wässrigen Lösung von Stärke
mit Glaskugeln bis zu einer Korngrösse von 2-4 µm gemahlen.
- 2,8 g: der Dispersion A und
- 5,8 g: der Dispersion B
werden zu einer Streichmasse verarbeitet und so auf ein Papier mit einem Flächengewicht von 50 g/m² aufgetragen, dass die aufgebrachte Streichmischung 2 g/m² Trockengewicht entspricht.

Durch Verwendung des Papiers in einem Faksimile-Gerät (Infotex 6510) entwickelt sich eine rotviolette Farbe.

## Patentansprüche

1. Methylenpyrrolin der Formel 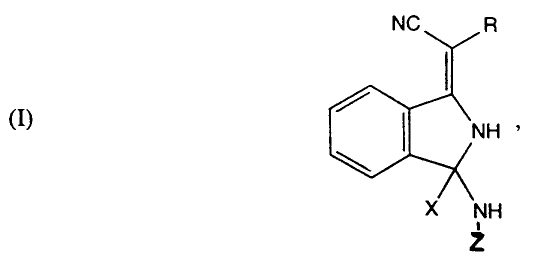 worin
X Indolyl, einen unsubstituierten oder bis zu dreifach gleich oder verschieden durch Halogen, Cyano, C₁-C₁₂-Alkyl, C₅-C₆-Cycloalkyl, C₁-C₈**-**Acyl, -NR₁R₂, -OR₃ oder -SR₃ substituierten Phenylrest,
R CN oder durch Hydroxy, Halogen, Nitro, Cyano, Acyl mit 1 bis 8 Kohlenstoffatomen, unsubstituiertes oder durch Halogen, Hydroxy, Cyano oder C₁-C₁₂-Alkoxy substituiertes C₁-C₁₂-Alkyl, oder C₁-C₁₂-Alkoxy,
Z C₁-C₁₂-Alkyl-CO- oder unsubstituiertes oder durch Halogen, Nitro, C₁-C₁₂-Alkyl oder C₁-C₁₂-Alkoxy substituiertes Benzoyl,
R₁, R₂ und R₃, unabhängig voneinander. Wasserstoff, unsubstituiertes oder durch Halogen, Hydroxy, Cyano oder C₁-C₁₂-Alkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₁₂-Alkyl, C₁-C₁₂-Alkoxy, C₁-C₁₂-Alkoxycarbonyl, -NX'X'' oder 4-NX'X''-Phenylamino ringsubstituiertes Phenylalkyl oder Phenyl,
X' und X'', unabhängig voneinander, Wasserstoff, C₁-C₁₂-Alkyl, Cyclohexyl, Benzyl oder Phenyl oder
R₁ und R₂ zusammen mit dem sie verbindenden Stickstoffatom einen fünf- oder sechsgliedrigen, vorzugsweise gesättigten, heterocyclischen Rest
bedeuten.

2. Mehylenpyrrolin gemäss Anspruch 1, worin
X 2-Methylindol-3-yl, N**-**C₁-C₈-Alkyl-2**-**mehylindol**-**3**-**yl, N-C₂-C₄-Alkanoyl**-**2-methylindol**-**3-yl, 2-Phenylindol**-**3**-**yl oder N-C₁-C₈-Alkyl-2**-**phenylindol**-**3**-**yl
bedeutet.

3. Pyrrolin gemäss Anspruch 1, worin
X einen unsubstituierten oder bis zu dreifach gleich oder verschieden durch Halogen, Cyano, C₁-C₁₂-Alkyl, C₅-C₆-Cycloalkyl, C₁-C₈-Acyl, -NR₁R₂, -OR₃ oder -SR₃ substituierten Phenylrest bedeutet.

4. Pyrrolin gemäss Anspruch 3, worin
X einen Phenylrest der Formel 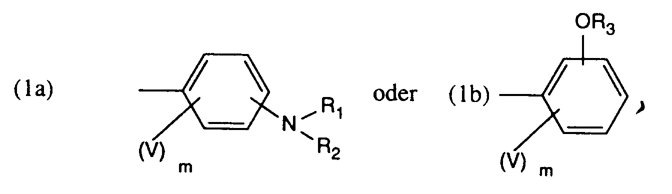 worin
R₁, R₂ und R₃, unabhängig voneinander, Wasserstoff, unsubstituiertes oder durch Halogen, Hydroxy, Cyano oder Niederalkoxy substituiertes Alkyl mit höchstens 12 Kohlenstoffatomen, Acyl mit 1 bis 8 Kohlenstoffatomen, Cycloalkyl mit 5 bis 10 Kohlenstoffatomen oder unsubstituiertes oder durch Halogen, Trifluormethyl, Cyano, C₁-C₁₂-Alkyl, C₁-C₁₂-Alkoxy, C₁-C₁₂-Alkoxycarbonyl, -NX'X'' oder 4-NX'X''-Phenylamino ringsubstituiertes Phenylalkyl oder Phenyl,
X' und X'', unabhängig voneinander, Wasserstoff, C₁-C₁₂-Alkyl, Cyclohexyl, Benzyl oder Phenyl oder
R₁ und R₂ zusammen mit dem sie verbindenden Stickstoffatom einen fünf oder sechsgliedrigen, vorzugsweise gesättigten, heterocyclischen Rest,
V Hydroxy, Halogen, C₁-C₁₂-Alkyl, C₁-C₁₂-Alkoxy, C₅-C₇-Cycloalkoxy, C₁-C₁₂-Acyloxy, unsubstituiertes oder im Phenylrest durch Halogen, Cyano, C₁-C₁₂-Alkyl oder C₁-C₁₂-Alkoxy substituiertes Benzyl, Phenyl, Benzyloxy, Phenoxy oder beim Phenylrest der Formel (1a) auch die Gruppe -NT₁T₂,
T₁ und T₂, unabhängig voneinander, Wasserstoff, C₁-C₁₂-Alkyl, C₅-C₁₀-Cycloalkyl, unsubstituiertes oder durch Halogen, Cyano, C₁-C₁₂-Alkyl oder C₁-C₁₂-Alkoxy substituiertes Benzyl, oder Acyl mit 1 bis 8 Kohlenstoffatomen und
T₁ auch unsubstituiertes oder durch Halogen, Cyano, C₁-C₁₂-Alkyl oder C₁-C₁₂-Alkoxy substituiertes Phenyl, und
m 0, 1 oder 2,
bedeutet.

5. Pyrrolin gemäss Anspruch 1 bis 4 worin
Z eine Acylgruppe der Formel Y'-CO- und
Y' C₃-C₈-Alkyl oder Phenyl bedeutet.

6. Pyrrolin gemäss einem der Ansprüche 1 bis 5, worin
R einen Rest der Formel 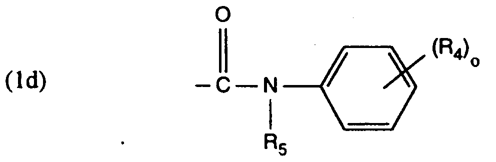
R₄ unabhängig voneinander Hydroxy, Halogen, Nitro, Cyano, Acyl mit 1 bis 8 Kohlenstoffatomen, unsubstituiertes oder durch Halogen, Hydroxy, Cyano oder C₁-C₁₂-Alkoxy substituiertes C₁-C₁₂-Alkyl, oder C₁-C₁₂-Alkoxy,
R₅ Wasserstoff oder C₁-C₁₂-Alkyl und
o 0, 1, 2 oder 3,
bedeutet.

7. Isoindolin gemäss Anspruch 1, der Formel 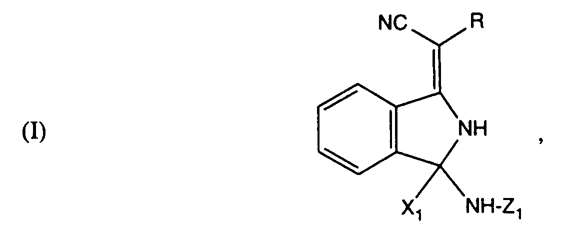 worin
Z₁ C₁-C₁₂-Alkyl-CO- oder unsubstituiertes oder durch Halogen, Nitro, C₁-C₁₂-Alkyl oder C₁-C₁₂-Alkoxy substituiertes Benzoyl und
X₁ einen 3-Indolylrest der Formel 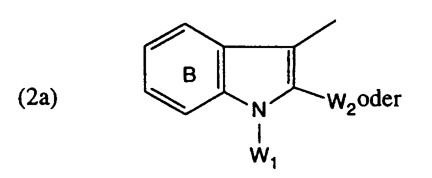 einen substituierten Phenylrest der Formel 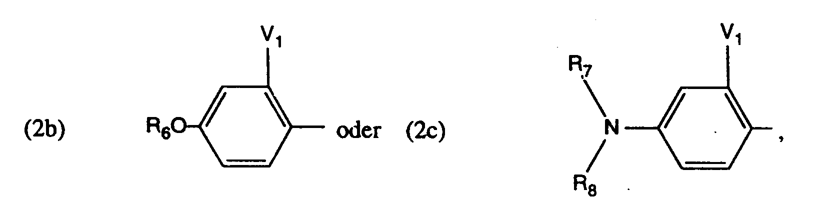
W₁ Wasserstoff, unsubstituiertes oder durch Cyano oder C₁-C₁₂-Alkoxy substituiertes C₁-C₈-Alkyl, Acetyl, Propionyl oder Benzyl,
W₂ Wasserstoff, C₁-C₁₂-Alkyl,
R₆, R₇ und R₈, unabhängig voneinander, je unsubstituiertes oder durch Hydroxy, Cyano oder C₁-C₁₂-Alkoxy substituiertes Alkyl mit höchstens 12 Kohlenstoffatomen, C₅-C₆-Cycloalkyl, Benzyl, Phenethyl oder Phenyl und R₆ auch Wasserstoff, oder (R₅ und R₆) zusammen mit dem sie verbindenden Stickstoffatom Pyrrolidino, Piperidino oder Morpholino,
V₁ Wasserstoff, Hydroxy, Halogen, C₁-C₁₂-Alkyl, C₁-C₈-Alkoxy, Benzyloxy oder die Gruppe -NT₃T₄,
T₃ und T₄, unabhängig voneinander, je Wasserstoff, C₁-C₁₂-Alkyl, C₁-C₁₂-Alkylcarbonyl oder unsubstituiertes oder durch Halogen, Methyl oder Methoxy substituiertes Benzoyl
R Cyano oder einen Rest der Formel 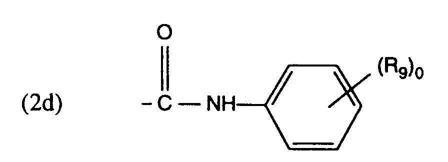
R₉ Halogen oder C₁-C₁₂-Alkyl,
o 0, 1 oder 2 bedeutet und
B nicht substituiert oder durch Halogen, C₁-C₁₂-Alkyl oder durch C₁-C₁₂-Alkylamino substituiert ist.

8. Isoindolin gemäss Anspruch 7, worin
X₁ einen 3-Indolylrest der Formel (2a),
W₁ Wasserstoff oder C₁-C₈-Alkyl,
W₂ Methyl oder Phenyl und
Z₁ C₃-C₈-Alkylcarbonyl,
bedeutet.

9. Dicyanoisoindolin gemäss Anspruch 1, der Formel

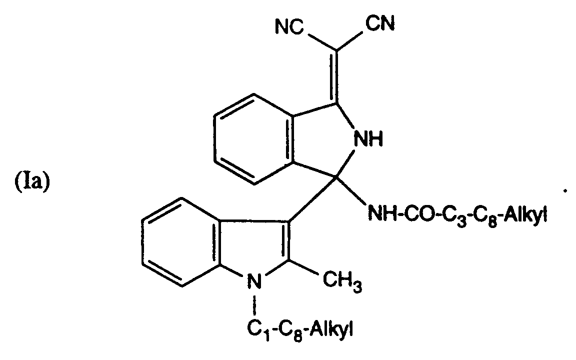

10. Dicyanoisoindolin gemäss Anspruch 1, der Formel 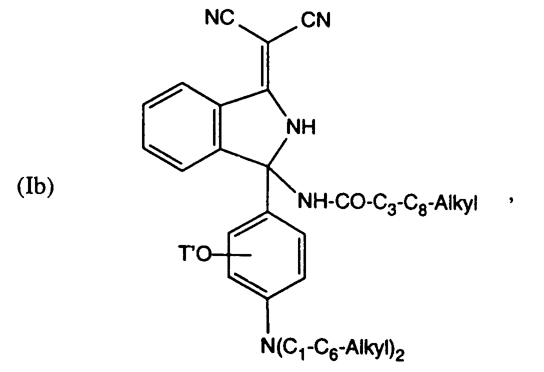 worin T' Wasserstoff oder C₁-C₆-Alkyl bedeutet.

11. Dicyanoisoindolin gemäss Anspruch 1, der Formel 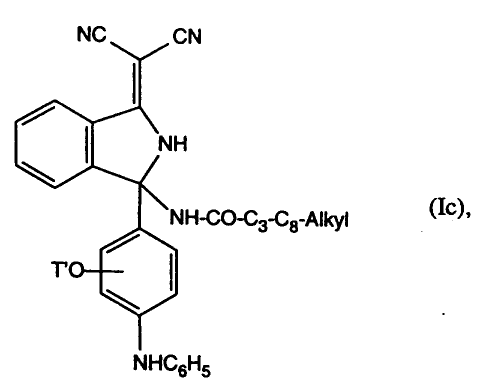 worin T Wasserstoff oder C₁-C₆-Alkyl bedeutet.

12. Isoindolin gemäss Anspruch 1, der Formel 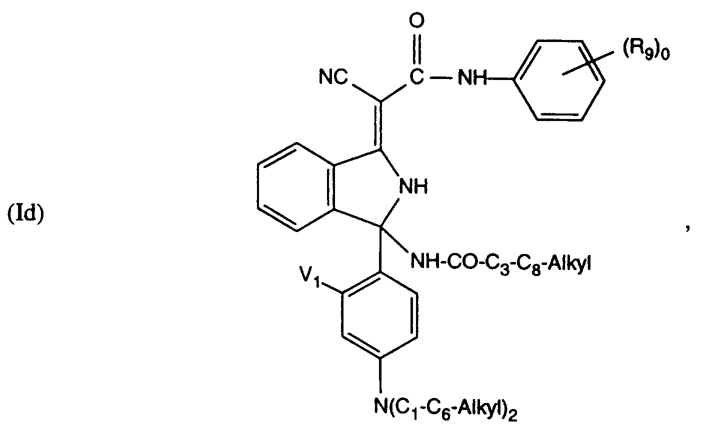 worin
R₉ Halogen oder C₁-C₁₂-Alkyl,
o 0, 1 oder 2 und
V₁ Wasserstoff oder Hydroxy
bedeutet.

13. Isoindolin gemäss Anspruch 1, der Formel 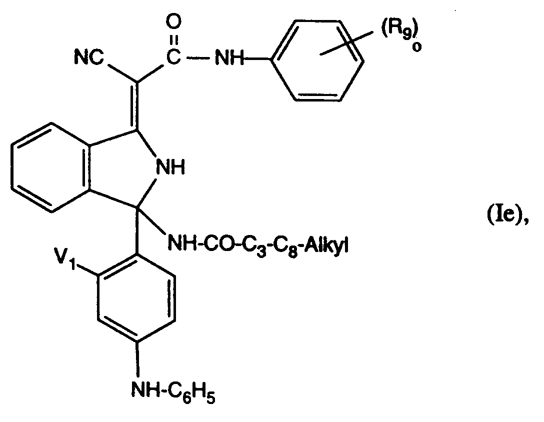 worin
R₉ Halogen, C₁-C₁₂-Alkyl oder C₁-C₁₂-Alkoxy,
o 0, 1 oder 2 und
V₁ Wasserstoff oder Hydroxy
bedeutet.

14. Verfahren zur Herstellung einer Verbindung gemäss einem der Ansprüche 1 bis 13, durch Umsetzung einer aromatischen oder heteroaromatischen Verbindung der Formel
X-H (II)
worin
X wie zuvor definiert ist, mit einem Pyrrolinderivat der Formel (III) 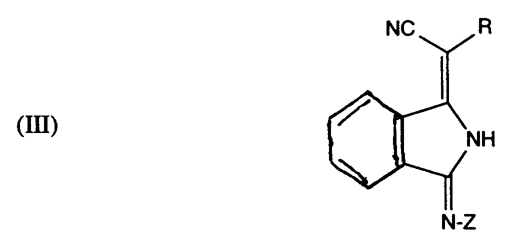 worin
R und Z wie in Anspruch 1 definiert sind.

## Claims

1. A methylenepyrroline of the formula 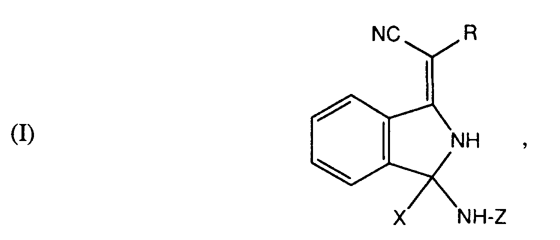 in which
X is indolyl or a phenyl radical which is unsubstituted or substituted by up to three identical or different substituents from the group consisting of halogen, cyano, C₁-C₁₂alkyl, C₅-C₆cycloalkyl, C₁-C₈acyl, -NR₁R₂, -OR₃ or -SR₃,
R is CN or is phenylcarbamoyl which is substituted by hydroxyl, halogen, nitro, cyano, acyl having 1 to 8 carbon atoms, unsubstituted or halogen-, hydroxyl-, cyano- or C₁-C₁₂alkoxy-substituted C₁-C₁₂alkyl, or C₁-C₁₂alkoxy,
Z is C₁-C₁₂alkyl-CO- or benzoyl which is unsubstituted or substituted by halogen, nitro, C₁-C₁₂alkyl or C₁-C₁₂alkoxy,
R₁, R₂ and R₃, independently of one another, are hydrogen, unsubstituted or halogen-, hydroxyl-, cyano- or C₁-C₁₂alkoxy-substituted alkyl having at most 12 carbon atoms, acyl having 1 to 8 carbon atoms, cycloalkyl having 5 to 10 carbon atoms or unsubstituted or halogen-, trifluoromethyl-, cyano-, C₁-C₁₂alkyl-, C₁-C₁₂alkoxy-, C₁-C₁₂alkoxycarbonyl-, -NX'X''- or 4-NX'X''-phenylamino-ring-substituted phenylalkyl or phenyl,
X' and X'', independently of one another, are hydrogen, C₁-C₁₂alkyl, cyclohexyl, benzyl or phenyl or
R₁ and R₂ together with the nitrogen atom linking them are a five- or six-membered, preferably saturated, heterocyclic radical.

2. A methylenepyrroline as claimed in claim 1, in which
X is 2-methylindol-3-yl, N-C₁-C₈alkyl-2-methylindol**-**3-yl, N-C₂**-**C₄alkanoyl-2-methylindol-3-yl, 2-phenylindol-3-yl or N-C₁-C₈alkyl-2-phenylindol-3-yl.

3. A pyrroline as claimed in claim 1, in which
X is a phenyl radical which is unsubstituted or substituted by up to three identical or different substituents from the group consisting of halogen, cyano, C₁-C₁₂alkyl, C₅-C₆cycloalkyl, C₁-C₈acyl, -NR₁R₂, -OR₃ or -SR₃.

4. A pyrroline as claimed in claim 3, in which
X is a phenyl radical of the formula 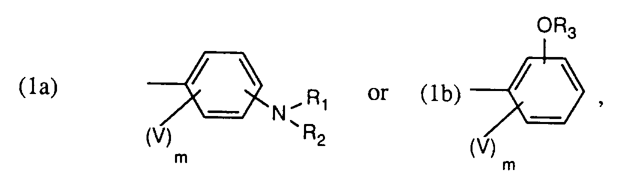 in which
R₁, R₂ and R₃, independently of one another, are hydrogen, unsubstituted or halogen-, hydroxyl-, cyano- or C₁-C₁₂alkoxy-substituted alkyl having at most 12 carbon atoms, acyl having 1 to 8 carbon atoms, cycloalkyl having 5 to 10 carbon atoms or unsubstituted or halogen-, trifluoromethyl-, cyano-, C₁-C₁₂alkyl-, C₁-C₁₂alkoxy-, C₁-C₁₂alkoxycarbonyl-, -NX'X''**-** or 4-NX'X''-phenylamino-ring-substituted phenylalkyl or phenyl,
X' and X'', independently of one another, are hydrogen, C₁-C₁₂alkyl, cyclohexyl, benzyl or phenyl or
R₁ and R₂ together with the nitrogen atom linking them are a five- or six-membered, preferably saturated, heterocyclic radical,
V is hydroxyl, halogen, C₁-C₁₂alkyl, C₁-C₁₂alkoxy, C₅-C₇cycloalkoxy, C₁-C₁₂acyloxy, benzyl, phenyl, benzyloxy or phenoxy each of which is unsubstituted or substituted in the phenyl radical by halogen, cyano, C₁-C₁₂alkyl or C₁-C₁₂alkoxy, or, in the case of the phenyl radical of the formula (1a), also the group -NT₁T₂,
T₁ and T₂, independently of one another, are hydrogen, C₁-C₁₂alkyl, C₅-C₁₀cycloalkyl, unsubstituted or halogen-, cyano-, C₁-C₁₂alkyl- or C₁-C₁₂alkoxy-substituted benzyl, or acyl having 1 to 8 carbon atoms and
T₁ is also unsubstituted or halogen-, cyano-, C₁-C₁₂alkyl- or C₁-C₁₂alkoxy-substituted phenyl, and
m is 0, 1 or 2.

5. A pyrroline as claimed in claims 1 to 4, in which
Z is an acyl group of the formula Y'-CO- and
Y' is C₃-C₈alkyl or phenyl.

6. A pyrroline as claimed in any one of claims 1 to 5, in which
R is a radical of the formula 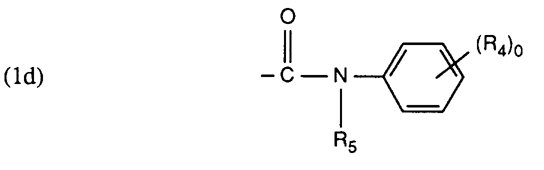 in which
each R₄, independently of the others, is hydroxyl, halogen, nitro, cyano, acyl having 1 to 8 carbon atoms, unsubstituted or halogen-, hydroxyl-, cyano- or C₁-C₁₂alkoxy-substituted C₁-C₁₂alkyl, or C₁-C₁₂alkoxy,
R₅ is hydrogen or C₁-C₁₂alkyl and
o is 0, 1, 2 or 3.

7. An isoindoline as claimed in claim 1, of the formula 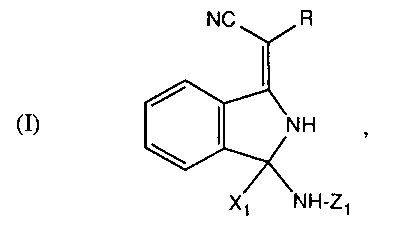 in which
Z₁ is C₁-C₁₂alkyl-CO- or benzoyl which is unsubstituted or substituted by halogen, nitro, C₁-C₁₂alkyl or C₁-C₁₂alkoxy and
X₁ is a 3-indolyl radical of the formula 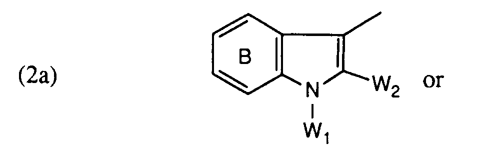 a substituted phenyl radical of the formula 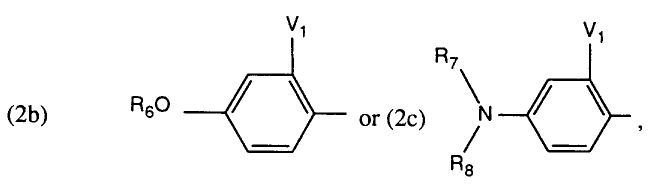
W₁ is hydrogen, unsubstituted or cyano- or C₁-C₁₂alkoxy-substituted C₁-C₈alkyl, acetyl, propionyl or benzyl,
W₂ is hydrogen, C₁-C₁₂alkyl,
R₆, R₇ and R₈, independently of one another, are each unsubstituted or hydroxyl-, cyano- or C₁-C₁₂alkoxy-substituted alkyl having at most 12 carbon atoms, C₅**-**C₆cycloalkyl, benzyl, phenethyl or phenyl and R₆ is also hydrogen, or (R₅ and R₆) together with the nitrogen atom linking them are pyrrolidino, piperidino or morpholino,
V₁ is hydrogen, hydroxyl, halogen, C₁-C₁₂alkyl, C₁-C₈alkoxy, benzyloxy or the group -NT₃T₄,
T₃ and T₄, independently of one another, are each hydrogen, C₁-C₁₂alkyl, C₁-C₁₂alkylcarbonyl or unsubstituted or halogen-, methyl- or methoxy-substituted benzoyl
R is cyano or a radical of the formula 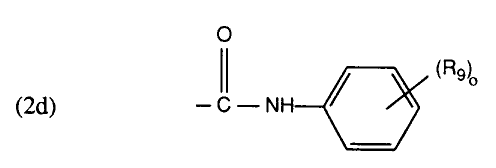
R₉ is halogen or C₁-C₁₂alkyl,
o is 0, 1 or 2 and
B is unsubstituted or substituted by halogen, C₁-C₁₂alkyl, or by (C₁-C₁₂alkyl)amino.

8. An isoindoline as claimed in claim 7, in which
X₁ is a 3-indolyl radical of the formula (2a),
W₁ is hydrogen or C₁-C₈alkyl,
W₂ is methyl or phenyl and
Z₁ is C₃-C₈alkylcarbonyl.

9. A dicyanoisoindoline as claimed in claim 1, of the formula

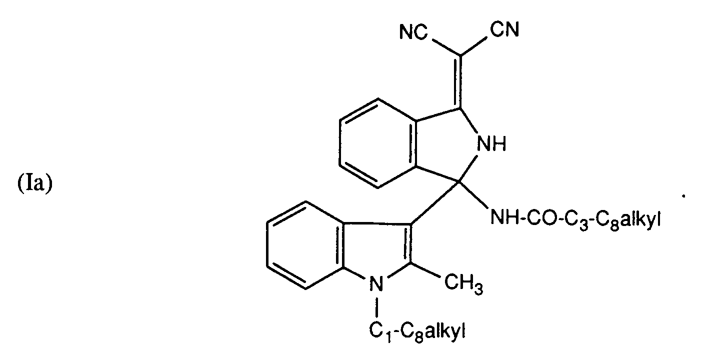

10. A dicyanoisoindoline as claimed in claim 1, of the formula 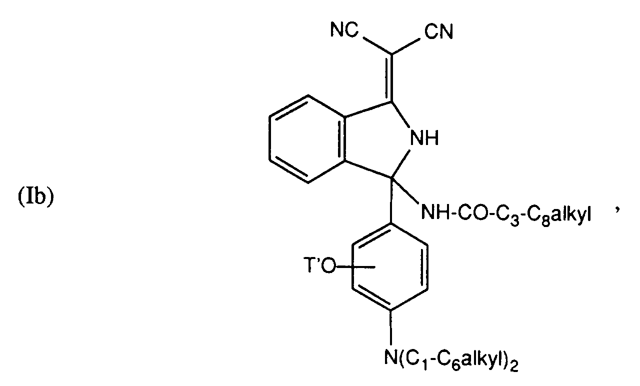 in which T' is hydogen or C₁-C₆alkyl.

11. A dicyanoisoindoline as claimed in claim 1, of the formula 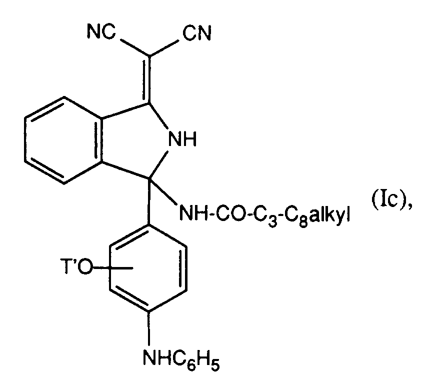 in which T' is hydrogen or C₁-C₆alkyl.

12. An isoindoline as claimed in claim 1, of the formula 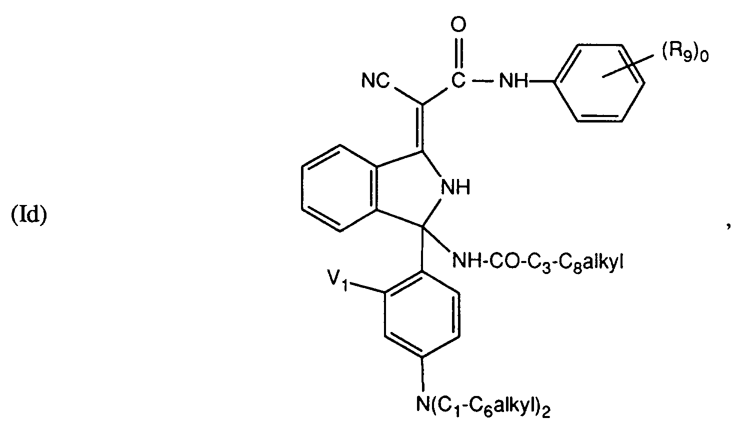 in which
R₉ is halogen or C₁-C₁₂alkyl,
o is 0, 1 or 2 and
V₁ is hydrogen or hydroxyl.

13. An isoindoline as claimed in claim 1, of the formula 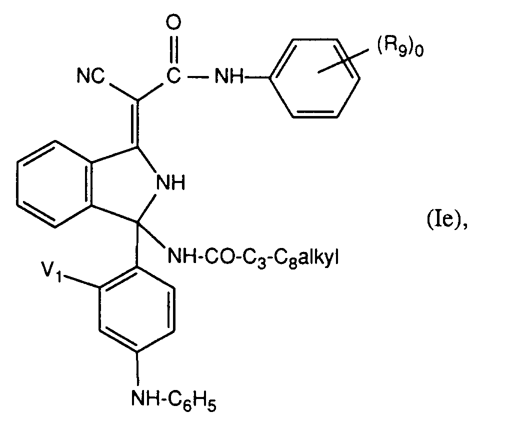 in which
R₉ is halogen, C₁-C₁₂alkyl or C₁-C₁₂alkoxy,
o is 0, 1 or 2 and
V₁ is hydrogen or hydroxyl.

14. A process for the preparation of a compound as claimed in any one of claims 1 to 13, which comprises reacting an aromatic or heteroaromatic compound of the formula
X-H (II)
in which
X is as defined above, with a pyrroline derivative of the formula (III) 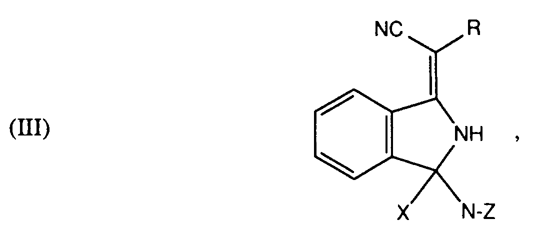 in which
R and Z are as defined in claim 1.

## Revendications

1. Méthylènepyrroline de formule 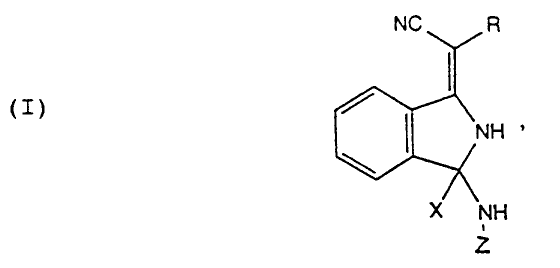 dans laquelle
X représente un reste indolyle, un reste phényle non substitué ou substitué jusqu'à 3 fois par des substituants, identiques ou différents, halogène, cyano, alkyle en C₁-C₁₂, cycloalkyle en C₅-C₆, acyle en C₁-C₈, -NR₁R₂, -OR₃ ou -SR₃,
R représente des groupes CN ou acyle comportant 1 à 8 atomes de carbone substitué par des substituants hydroxy, halogène, nitro, cyano, alkyle en C₁-C₁₂ non substitué ou substitué par halogène, hydroxy, cyano ou alkoxy en C₁-C₁₂, ou alkoxy en C₁-C₁₂,
Z représente (alkyl en C₁-C₁₂)-CO- ou benzoyle non substitué ou substitué par des substituants halogène, nitro, alkyle en C₁-C₁₂ ou alkoxy en C₁-C₁₂,
R₁, R₂ et R₃ représentent, indépendamment les uns des autres, un atome d'hydrogène, des groupes cycloalkyle avec 5 à 10 atomes de carbone, acyle avec 1 à 8 atomes de carbone, alkyle avec 12 atomes de carbone au maximum non substitué ou substitué par des substituants halogène, hydroxy, cyano ou alkoxy inférieur, ou représente phényle ou phénylalkyle non substitué ou substitué sur le cycle par des substituants halogène, trifluorométhyle, cyano, alkyle en C₁-C₁₂, alkoxy en C₁-C₁₂, alkoxycarbonyle en C₁-C₁₂, -NX'X''- ou 4-NX'X''-phénylamino,
X' et X'' représentent, indépendamment l'un de l'autre, un atome d'hydrogène, des groupes alkyle en C₁-C₁₂, cyclohexyle, benzyle ou phényle ou
R₁ et R₂ forment, ensemble avec l'atome d'azote qui les relie, un reste hétérocyclique de 5 à 6 chaînons, de préférence saturé.

2. Méthylènepyrroline selon la revendication 1, où
X représente 2**-**méthylindol-3**-**yle, N**-**(alkyl en C₁-C₈)**-**2**-**méthylindol**-**3**-**yle, N**-**(alcanoyl en C₂-C₄)-2-méthylindol**-**3**-**yle, 2**-**phénylindol**-**3**-**yle ou N**-**(alkyl en C₁**-**C₈)**-**2**-**phénylindol**-**3**-**yle.

3. Pyrroline selon la revendication 1, où
X représente un reste phényle non substitué ou substitué une à trois fois par des substituants, identiques ou différents, halogène, cyano, alkyle en C₁-C₁₂, cycloalkyle en C₅-C₆, acyle en C₁-C₈, -NR₁R₂, -OR₃ ou -SR₃.

4. Pyrroline selon la revendication 3, où
X représente un reste phényle de formule 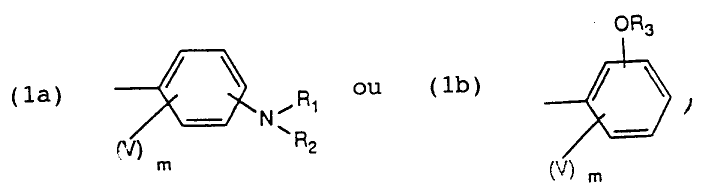 où
R₁, R₂ et R₃ représentent, indépendamment les uns des autres, de préférence un atome d'hydrogène, des groupes cycloalkyle avec 5 à 10 atomes de carbone, acyle avec 1 à 8 atomes de carbone, alkyle avec au maximum 12 atomes de carbone non substitué ou substitué par des substituants halogène, hydroxy, cyano ou alkoxy en C₁-C₁₂, ou représente phényle ou phénylalkyle non substitué ou substitué sur le cycle par des substituants halogène, trifluorométhyle, cyano, alkyle en C₁-C₁₂, alkoxy en C₁-C₁₂, (alkoxy en C₁**-**C₁₂)carbonyle, -NX'X''- ou 4-NX'X''-phénylamino,
X' et X'' représentent, indépendamment l'un de l'autre, un atome d'hydrogène, des groupes alkyle en C₁-C₁₂, cyclohexyle, benzyle ou phényle ou
R₁ et R₂ forment, ensemble avec l'atome d'azote qui les relie, un reste hétérocyclique de 5 à 6 chaînons, de préférence saturé,
V représente des groupes hydroxy, halogène, alkyle en C₁-C₁₂, alkoxy en C₁-C₁₂, cycloalkoxy en C₅-C₇, acyloxy en C₁-C₁₂, phénoxy, benzyloxy, phényle ou benzyle non substitué ou substitué sur le reste phényle par des substituants halogène, cyano, alkyle en C₁-C₁₂ ou alkoxy en C₁-C₁₂, ou dans le cas du reste phényle de formule (1a) aussi le groupe -NT₁T₂,
T₁ et T₂ représentent, indépendamment l'un de l'autre, un atome d'hydrogène, des groupes alkyle en C₁-C₁₂, cycloalkyle en C₅-C₁₀, benzyle non substitué ou substitué par des substituants halogène, cyano, alkyle en C₁-C₁₂ ou alkoxy en C₁-C₁₂, ou représente acyle avec 1 à 8 atomes de carbone et
T₁ représente aussi phényle non substitué ou substitué par des substituants halogène, cyano, alkyle en C₁-C₁₂ ou alkoxy en C₁-C₁₂, et
m vaut 0, 1 ou 2.

5. Pyrroline selon la revendication 1 à 4, dans laquelle
Z représente un groupe acyle de formule Y'-CO- et
Y' représente des groupes alkyle en C₃-C₈ ou phényle.

6. Pyrroline selon l'une des revendications 1 à 5, où
R représente un reste de formule 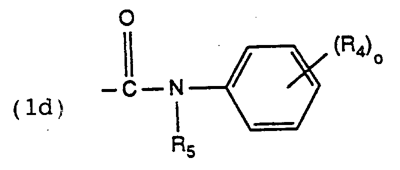
R₄ représentent, indépendamment l'un de l'autre, des groupes hydroxy, halogène, nitro, cyano, acyle avec 1 à 8 atomes de carbone, ou alkyle en C₁-C₁₂ non substitué ou substitué par des groupes halogène, hydroxy, cyano ou alkoxy en C₁-C₁₂, ou alkoxy en C₁-C₁₂,
R₅ représente un atome d'hydrogène ou un groupe alkyle en C₁-C₁₂ et
o vaut 0, 1, 2 ou 3.

7. Isoindoline selon la revendication 1, de formule 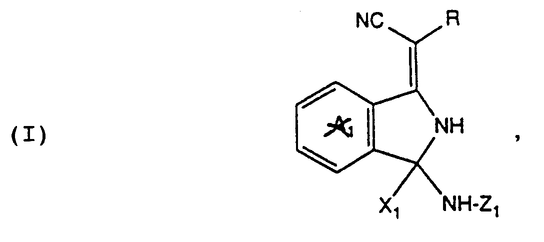 dans laquelle
Z₁ représente un groupe (alkyl en C₁-C₁₂)-CO- ou benzoyle non substitué ou substitué par des substituants halogène, nitro, alkyle en C₁-C₁₂ ou alkoxy en C₁-C₁₂,
X₁ représente un reste 3**-**indolyle de formule 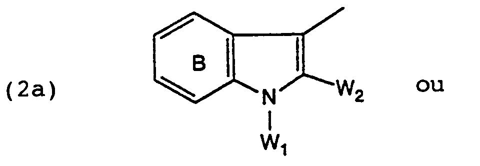 un reste phényle substitué de formule 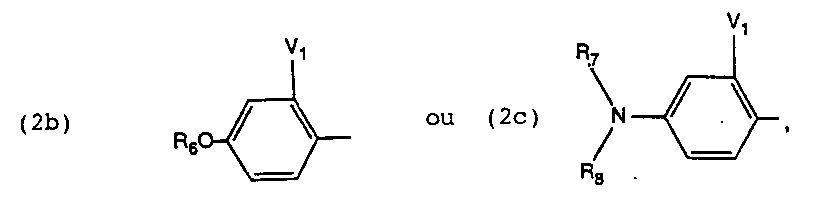
W₁ représente un atome d'hydrogène, des groupes benzyle, propionyle, acétyle ou alkyle en C₁-C₈ non substitué ou substitué par des substituants cyano ou alkyle en C₁-C₁₂,
W₂ représente un atome d'hydrogène, un groupe alkyle en C₁-C₁₂,
R₆, R₇ et R₈ représentent chacun, indépendamment l'un de l'autre, un groupe phényle, phénéthyle, benzyle, cycloalkyle en C₅-C₆ ou alkyle avec au maximum 12 atomes de carbone à chaque fois non substitué ou substitué par des substituants hydroxy, cyano ou alkoxy en C₁-C₁₂, et R₆ représente aussi un atome d'hydrogène, ou (R₆ et R₇) représentent, conjointement avec l'atome d'azote qui les relie, des groupes pyrrolidino, pipéridino ou morpholino,
V₁ représente un atome d'hydrogène des groupes hydroxy, halogène, alkyle en C₁-C₁₂, alkoxy en C₁-C₈, benzyloxy ou le groupe -NT₃T₄,
T₃ et T₄ représentent chacun, indépendamment l'un de l'autre, un atome d'hydrogène, des groupes alkyle en C₁-C₁₂, (alkyl en C₁-C₁₂)carbonyle ou benzoyle non substitué ou substitué par des substituants halogène, méthyle ou méthoxy,
R représente un groupe cyano ou un reste non substitué ou substitué de formule 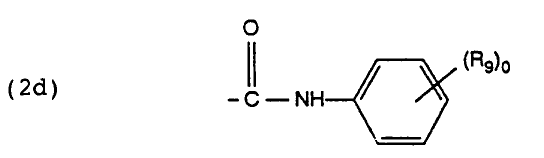
R₉ représente un atome d'halogène ou un groupe alkyle en C₁-C₁₂,
o vaut 0, 1 ou 2 et
B est non substitué ou substitué par des substituants halogène, alkyle en C₁-C₁₂, tel que méthyle ou isopropyle, ou par di-(alkyl en C₁-C₁₂)-amino, tel que diéthylamino.

8. Isoindoline selon la revendication 7, où
X₁ représente un reste 3-indolyle de formule (2a),
W₁ représente un atome d'hydrogène ou un groupe alkyle en C₁-C₈,
W₂ représente les groupes méthyle ou phényle et
Z₁ représente un groupe (alkyl en C₃**-**C₈)carbonyle.

9. Dicyanoisoindoline selon la revendication 1, de formule

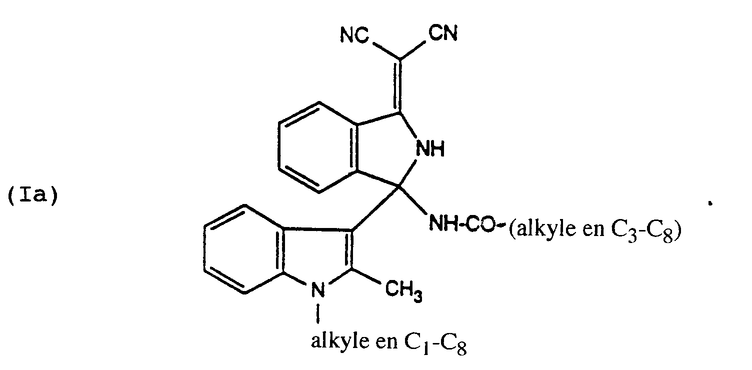

10. Dicyanoisoindoline selon la revendication 1, de formule 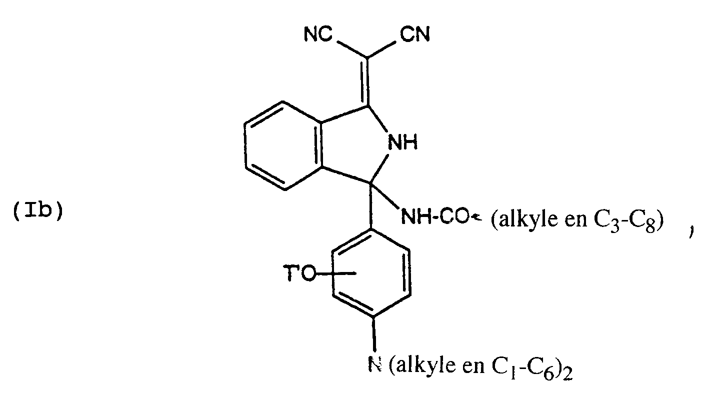 dans laquelle T' représente un atome d'hydrogène ou un groupe alkyle en C₁-C₆.

11. Diocyanoisoindoline selon la revendication 1, de formule 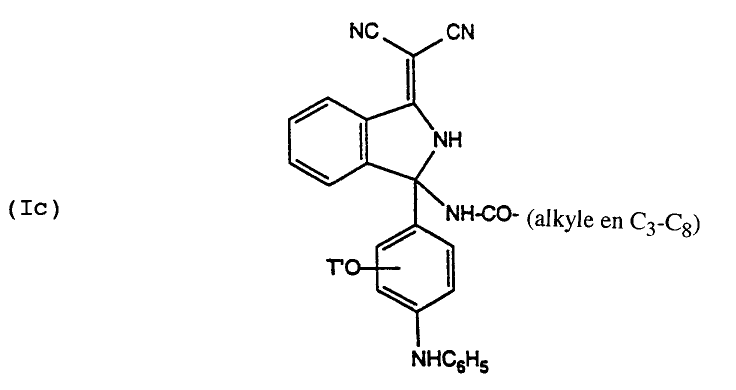 dans laquelle T' représente un atome d'hydrogène ou un groupe alkyle en C₁-C₆.

12. Isoindoline selon la revendication 1, de formule 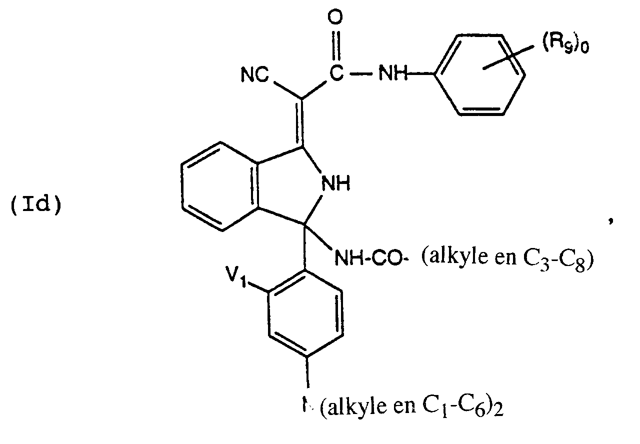 dans laquelle
R₉ représente un atome d'halogène ou un groupe alkyle en C₁-C₁₂,
o vaut 0, 1 ou 2 et
V₁ représente un atome d'hydrogène ou un groupe hydroxy.

13. Isoindoline selon la revendication 1, de formule 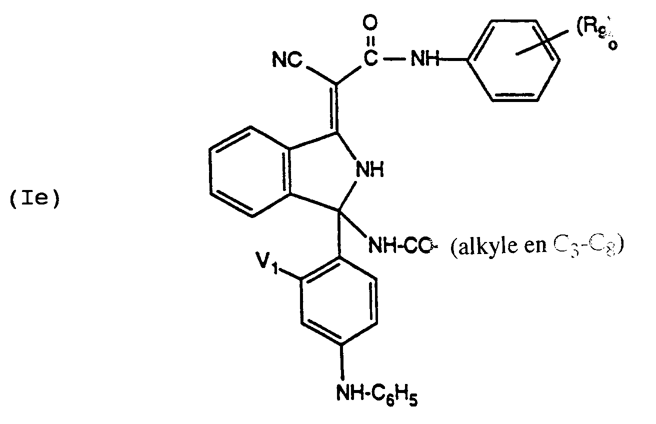 dans laquelle
R₉ représente un atome d'halogène, des groupes alkyle en C₁-C₁₂ ou alkoxy en C₁-C₁₂,
o vaut 0, 1 ou 2 et
V₁ représente un atome d'hydrogène ou un groupe hydroxy.

14. Procédé pour la préparation d'un composé selon l'une des revendications 1 à 13, par transformation d'un composé aromatique ou hétéroaromatique de formule
X-H (II)
où
X est défini comme ci-dessus, avec un dérivé de pyrroline de formule (III) 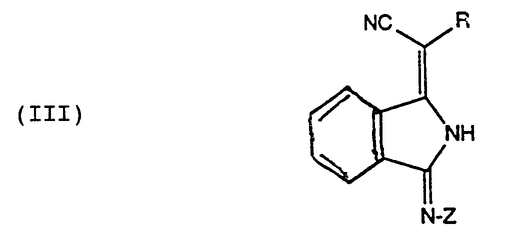 où
R et Z sont défini à la revendication 1.
